(19) Europäisches Patentamt
European Patent Office
Office européen des brevets

(11) EP 3 978 006 B1

(12) EUROPÄISCHE PATENTSCHRIFT

(45) Veröffentlichungstag und Bekanntmachung des Hinweises auf die Patenterteilung:
**05.11.2025 Patentblatt 2025/45**

(21) Anmeldenummer: **21155420.9**

(22) Anmeldetag: **05.02.2021**

(51) Internationale Patentklassifikation (IPC):
***A61K 36/48*** (2006.01) ***A61K 31/12*** (2006.01)
***A61K 31/525*** (2006.01) ***A61K 31/593*** (2006.01)
***A61K 33/04*** (2006.01) ***A61K 33/30*** (2006.01)
***A61K 45/06*** (2006.01) ***A61K 47/24*** (2006.01)
***A61P 39/06*** (2006.01) ***A61P 15/12*** (2006.01)
***A61K 9/00*** (2006.01)

(52) Gemeinsame Patentklassifikation (CPC):
(C-Sets verfügbar)
**A61K 36/48; A61K 9/0053; A61K 31/12; A61K 31/525; A61K 31/593; A61K 33/04; A61K 33/30; A61K 45/06; A61K 47/24; A61P 15/12; A61P 39/06** (Forts.)

(54) **ZUSAMMENSETZUNG MIT OPTIMIERTEM LEKTINGEHALT UND IHRE VERWENDUNG**
COMPOSITION WITH OPTIMIZED LECTIN CONTENT AND ITS USE
COMPOSITION AYANT UN TAUX DE LECTINE OPTIMISÉ ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.10.2020 EP 20199840**

(43) Veröffentlichungstag der Anmeldung:
**06.04.2022 Patentblatt 2022/14**

(73) Patentinhaber: **YACARE GmbH**
**79261 Gutach (DE)**

(72) Erfinder: **Müller, Georg**
**79263 Simonswald (DE)**

(74) Vertreter: **Hoefer & Partner Patentanwälte mbB**
**Pilgersheimer Straße 20**
**81543 München (DE)**

(56) Entgegenhaltungen:
**EP-A1- 2 106 705** **EP-A1- 2 218 456**
**DE-A1- 102010 033 458**

- **ALENCAR V B M ET AL: "Lectin of Pisum arvense seeds induces in-vivo and in-vitro neutrophil migration", vol. 57, no. 3, 28 February 2005 (2005-02-28), pages 375 - 381, XP009527619, ISSN: 0022-3573, Retrieved from the Internet <URL:https://api.wiley.com/onlinelibrary/tdm/v1/articles/10.1211%2F0022357055579> [retrieved on 20100218], DOI: 10.1211/0022357055579**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



(Forts. nächste Seite)

(52) Gemeinsame Patentklassifikation (CPC): (Forts.)

C-Sets
**A61K 31/12, A61K 2300/00;**
**A61K 31/525, A61K 2300/00;**
**A61K 31/593, A61K 2300/00;**
**A61K 33/04, A61K 2300/00;**
**A61K 33/30, A61K 2300/00;**
**A61K 36/48, A61K 2300/00**

## Beschreibung

Die Erfindung betrifft eine Lektinzusammensetzung mit optimierter Dosis und ihre Verwendung zur Prophylaxe und Behandlung.

## Hintergrund der Erfindung

Lektine und ihre Verwendung in verschiedenen Anwendungen sind bekannt.

Die EP 2 106 705 B1 beschreibt die Verwendung von Lektinen, Enzymen und Selen zur begleitenden Therapie von Krebspatienten. Hier wird eine hohe Dosis an Lektinen verwendet, wobei die Tagesdosis im Bereich von mindestens 15 bis 20 mg liegt.

Die EP 2 218 456 B1 beschreibt die Verwendung von Lektinen, Enzymen und Selen zur begleitenden Therapie von Arzneimittelnebenwirkungen. Hier wird eine hohe Dosis an Lektinen verwendet, wobei die Tagesdosis im Bereich von mindestens 2 bis 100 mg liegt.

Die DE 10 2010 033458 A1 beschreibt eine Lektinzusammensetzung und deren Verwendung zur Prophylaxe oder/und Behandlung von Nebenwirkungen verschiedener Krebsbehandlungsmethoden/Arzneimittelnebenwirkungen oder einem östrogen-bedingten Beschwerdekomplex.

Allerdings wird sowohl in den Patentansprüchen eine sehr hohe Dosis - 5mg Lektin bzw. eine Tagesdosis von 20mg Lektin - als auch in der Beschreibung ein entsprechend hoher Lektingehalt offenbart. Weiterhin hebt die DE 10 2010 033458 A1 in der Beschreibung hervor, dass eine Darreichung in Form von Mizellen vorteilhaft ist. Ein Schwerpunkt liegt damit auf der Mizellisierung der Lektine zur besseren Aufnahme der Lektine.

Die DE 10 2010 033458 A1enthält somit keinerlei Informationen aufgrund derer der Fachmann die beanspruchte Erfindung in naheliegender Weise ableiten könnte.

In der EP 2218456 A1 wird wie in der DE 10 2010 033458 A1 eine entsprechend hohe Lektinmenge vorgeschlagen. Alle hier offenbarten Lektindosen liegen mit 5mg oder mehr weit über der beanspruchten Dosis gemäß der beanspruchten Erfindung.

Weiterhin kann der Fachmann aus der Tabelle auf Seite 8 entnehmen dass höhere Lektin-Konzentrationen zu besseren Behandlungsergebnissen in Pateinten führen. Die Ergebnisse aus dem Vergleich von Patentien-Gruppe V und Gruppe VI legen nahe die Lektindosis zu erhöhen, um eine bessere Wirksamkeit zu erzielen.

Für jeden der gemessenen Parameter wie in Tabelle A dargestellt, zeigt sich für den Fachmann, dass durch eine Dosiserhöhung eine verbesserte Wirksamkeit erreicht werden kann. Der Fachmann lernt daraus, dass er bei Dosisfindungsüberlegungen die Dosis erhöhen soll, um die Behandlungsergebnisse zu verbessern.

Somit kann auch die EP 2218456 A1 den beanspruchten Gegenstand nicht nahelegen und führt vom beanspruchten Erfindungsgegenstand weg.

Ein Problem bei der Verabreichung von Lektinpräparaten sind Unverträglichkeiten und Nebenwirkungen, die sich negativ auf die Compliance der Patienten auswirkt und somit den Therapieerfolg vermindert.

Es war deshalb eine Aufgabe der vorliegenden Erfindung die Nachteile des Standes der Technik zu vermeiden oder zumindest zu verringern.

Er war eine weitere Aufgabe der vorliegenden Erfindung eine Lektinzusammensetzung mit verbesserter Wirkung bereitzustellen.

Er war eine weitere Aufgabe der vorliegenden Erfindung eine Lektinzusammensetzung mit verminderten Nebenwirkungen bereitzustellen.

## Kurze Zusammenfassung der Erfindung

Die Erfindung ist gerichtet auf eine Zusammensetzung nach Anspruch 1 und eine Verwendung nach Anspruch 11.

Kurze Beschreibung der Figuren

Figur 1 und 5 beschreibt die Dosisabhängigkeit der Wirksamkeit bei Zusammensetzungen mit unterschiedlichem Lektingehalt gemäß der Erfindung (Fig. 1) im Vergleich zum Stand der Technik anhand von verschiedenen Parametern bzw. reduzierte Nebenwirkungen (Fig. 5); insbesondere wird aus Figur 1, die sich aus den Daten der Tabelle 1 ergibt, deutlich, dass eine Lektindosis von ca. 0,2 bis 0,04 mg Lektin zu verminderten Nebenwirkungen führt, aber gleichzeitig überraschender Weise auch zu einer verbesserten Wirksamkeit gegenüber einer Lektindosis von 20 mg führt. Im Vergleich zu bekannten Lektinpräparaten und Anwendungen des Standes der Technik wurden erfindungsgemäß Lektinverdünnungen von ca. 1:100 bis 1:500 und teilweise bis 1:1250 verwendet, die sich nicht nur durchverminderte Nebenwirkungen, sondern durch eine verbesserte Wirksamkeit auszeichneten. Verdünnungen von 1:100 bzw. 1:1250 zeigten diese Verbesserungen dann nicht mehr. Es wird also mit der Erfindung vorteilhafter Weise eine Verminderung von Nebenwirkungen erzielt bei gleichzeitiger Wirksamkeitssteigerung, was der Fachmann nicht erwarten konnte. Außerdem haben die Erfinder somit vorteilhafterweise und überraschend einen optimierten Wirkkorridor bei gleichzeitiger Minimierung von Nebenwirkungen gefunden.

Figur 2 beschreibt die Ergebnisse eines "Burst"-Bluttestes mit Proben von 5 Probanden, wobei eine Dosis von unterschiedlichen Lektingehalten verwendet wurden; 1:100 in der Figur entspricht einer Lektindosis von 0.2 mg, 1:500 entspricht einer Lektindosis von 0.04 mg, 1:1000 entspricht einer Lektindosis von 0.02 mg und 1:1250 entspricht einer Lektindosis von 0.016 mg. Dieser in vitro Bluttest bestätigt einerseits die Ergebnisse des klinischen Tests, der in Figur 1 dargestellt ist, dass eine Lektindosis von ca. 0,02 bis 0,04 mg Lektin eine verbesserte Wirksamkeit bewirkt. Andererseits vermindert sich die Wirksamkeit bei weiterer Reduzierung der Lektindosis wieder (siehe 0,02 bzw. 0,016 mg Lektindosis). Die Verminderung in der Wirksamkeit bei weiterer Verminderung des Lektingehaltes zeigt aber andererseits auch die Validität diese *in vitro* Testes. Die Nummern 1, 2, 3, 4 bzw. 5 bezeichnen dabei die unterschiedlichen Proben wie auch aus den entsprechenden Tabelle 2 ersichtlich ist.

Figur 3 und Figur 4 stellen die Ergebnisse eines LTT bzw. NK/Modulatoren-Test dar, wobei entsprechend Figur 2 dieselben unterschiedlichen Lektinmengen der Probanden 1, 2, 3, 4 bzw. 5 (siehe Tabellen 3 und 4) untersucht wurden; die Figuren 3 und 4 zeigen entsprechende Ergebnisse wie Figur 2.

Alle drei *in vitro* Tests bestätigen damit nicht nur die Ergebnisse der in Figur 1 dargestellten klinischen Untersuchung, sondern deren Ergebnisse bei weiterer Verminderung der Lektinmenge belegen auch deren Validität an sich. Es konnte so mittels zweier Untersuchungsansätze, d.h. im klinischen Versuch als auch in *in vitro* Tests (immunologische Untersuchungen), ein optimierter Korridor der Wirksamkeit bei gleichzeitig verminderten Nebenwirkungen für Lektinanwendungen gemäß der Erfindung zweifelsfrei gefunden wurden.

## Ausführliche Beschreibung der Erfindung

Die Erfindung ist gerichtet auf eine Zusammensetzung nach Anspruch 1 und eine Verwendung nach Anspruch 11.

In einem Aspekt betrifft die Offenbarung eine Zusammensetzung a. zur Verwendung zur Prophylaxe oder/und Behandlung von (AHT) Aromatase-Hemmer-, Antiöstrogen- oder/und GnRH-Analoga - bedingten unerwünschten Arzneimittelnebenwirkungen umfassend Austrocknen des gesamten Schleimhautkomplexes umfassend Augen-, Nasen-, Mund-, Rachen-, Vaginalschleimhäute sowie der des Magen-Darm-Traktes, Gelenkschmerzen, insbesondere morgendlicher Anlaufschmerz und Hitzewallungen oder b. zur Verwendung zur Prophylaxe oder/und Behandlung von Chemo- oder Strahlentherapie induzierten Arzneimittelnebenwirkungen umfassend Übelkeit, Erbrechen/Durchfall, trockene Schleimhäute, Gelenkbeschwerden, Müdigkeit/Schwäche, Mundtrockenheit oder c. zur Verwendung zur Prophylaxe oder/und Behandlung von natürlichem Hormondefizit bedingt durch die Wechseljahre, umfassend Hitzewallungen, Augentrockenheit, Trockenheit der Vaginalschleimhaut sowie Gelenkprobleme und einer erhöhten Rate von Infektionen im urogenitalen Bereich, Gewichtszunahme, übermäßige Nervosität sowie Empfindlichkeit, Müdigkeit, psychische Probleme wie Angstzustände, Schlafstörungen und depressive Zustände.

Überraschender Weise konnte gezeigt werden, dass durch die Bereitstellung einer erfindungsgemäßen Lektinzusammensetzung nicht nur die Nebenwirkungen vermindert, sondern auch die Wirksamkeit der Wirksubstanz Lektin erhöht werden konnte. Dies ist insbesondere aus den Beispielen und hier enthaltenen Daten wie in den Figuren und Tabellen dargestellt ersichtlich.

Im Gegensatz zu bekannten Lektinzusammensetzungen beschreibt die vorliegende Offenbarung eine Zusammensetzung mit einem um mehrere Verdünnungsstufen verminderten Lektingehalt. Es konnte überraschender Weise gefunden werden, dass ein Lektingehalt bzw. eine Tagesdosis an Linsenlektin (Lens culinaris) von 0,5 bis 0,08 mg und gegebenenfalls weitere Zusatz- und Hilfsstoffe und wobei die Zusammensetzung eine pharmazeutische Zusammensetzung, ein diätetisches Lebensmittel, ein Lebensmittel für die bilanzierte Diät, ein Nahrungsergänzungsmittel oder ein Medizinprodukt ist, nicht nur zu einer Verminderung der Nebenwirkungen führt, sondern dass in diesem Lektinbereich überraschender Weise eine Wirksamkeitssteigerung erzielt werden kann.

Es war nicht zu erwarten gewesen und damit überraschend, dass man bei Verminderung der Lektindosis die Nebenwirkungen vermindern kann und gleichzeitig eine Wirkungssteigerung erzielen könnte. Damit stellt die Erfindung vorteilhafter Weise eine Lektinzusammensetzung zur Verfügung, die nicht nur den Vorteil einer Nebenwirkungsverminderung und damit besserer Compliance der Pateinten erreichen kann, sondern eine Lektinzusammensetzung, die vorteilhafter Weise eine verbesserte Wirkung zeigt.

Die bekannten Veröffentlichungen, wie z.B. EP 2 106 705 B1 oder EP 2 218 456 B1, weisen einen vielfach höheren Lektingehalt auf. Damit verbunden sind die bekannten Nebenwirkungen von pharmazeitischen oder diätetischen Lebensmitteln. Es war nunmehr überraschend und unerwartet, dass eine Verminderung des Lektingehaltes in der Zusammensetzung zu einer verbesserten Wirksamkeit führen würde.

Weiterhin wirkt sich die verbesserte Wirksamkeit sowie die verminderten Nebenwirkungen gleichermaßen positiv auf die Compliance der Patienten aus und steigert somit nochmals den Behandlungserfolg.

In einer bevorzugten Ausführungsform kann die Zusammensetzung weiterhin mindestens eine Selenverbindung und/oder mindestens ein proteolytisches Enzym oder/und Vitamin D oder/und Vitamin B2 oder/und Zink oder/und Curcumin umfassen.

Diese zusätzlichen Komponenten in der Zusammensetzung können aus hierfür geeigneten Substanzen ausgewählt werden, wobei das Selen ein Natrium-Selenit, ein Kalium-Selenit, ein Magnesium-Selenit, ein Ammonium-Selenit oder/und eine vergleichbare Selenverbindung sein kann.

Die Tagesdosis der Selenverbindung oder einer äquimolaren Menge einer anderen Selenverbindung beträgt vorzugsweise mindestens 25-500 $\mu$g, vorzugsweise 50-300 $\mu$g, der Selenverbindung.

Weiterhin kann die Zusammensetzung ein proteolytisches Enzym enthalten, wobei das proteolytische Enzym ein Papain, ein Bromelain, ein Trypsin oder/und ein Chymotrypsin ist. Die Tagesdosis der proteolytischen Enzyme beträgt vorzugsweise mindestens 10-1000 mg oder 50-5000 FIP-Einheiten, vorzugsweise 20 - 1000 mg oder 100-5000 FIP-Einheiten.

In einem weiteren Aspekt der Offenbarung kann die Zusammensetzung wie hierin beschrieben weiteren Zusatz- und Hilfsstoffe enthalten, wobei diese ausgewählt sind aus der Gruppe bestehend aus Phospholipiden, vorzugsweise Phosphoglyceride, Mizellstabilisatoren, vorzugsweise ausgewählt aus der Gruppe bestehend aus Carnitin oder/und einem physiologisch verträglichen Carnitin-Derivat oder/und Polyol(en), oder/und Radikalfängern, vorzugsweise ausgewählt aus der Gruppe bestehend aus Ascorbinsäure oder/und Alkali- oder/und Erdalkalisalze der Ascorbinsäure oder/und Ester einer oder mehrerer organischer Säuren mit Ascorbinsäure.

Die Zusammensetzung wie hierin beschrieben kann dabei in jeder geeigneten Form formliert sein, wobei die Zusammensetzung zubereitet ist als eine Tablette, eine Kautablette, eine Retardtablette, eine Kapsel, ein Dragee, ein Pulver, ein Granulat, ein Lyophilisat, eine Suspension oder als wässrige oder ölige Lösung, Emulsion, Nanoemulsion, Nanodispersionssystem.

In einem weiteren Aspekt kann das Lektin emulgiert vorliegt in Vesikeln, Mischmizellen, als Mikroemulsion, als nanodisperses System oder Nanoemulsion, oder als flüssige, wässrige, mizellare Emulsion oder als flüssige, wässrig-ölige, mizellare Emulsion.

Dabei haben die Mizellen einen mittleren Durchmesser von 3 bis 300nm, vorzugsweise von 5 bis 250nm, besonders bevorzugt von 10 bis 120nm, mehr bevorzugt von 50 bis 80nm.

Der pH wird wie erforderlich und in Abstimmung mit den anderen Komponenten in geeigneter Weise auf einen geeigneten Wert eingestellt, wobei die Emulsion der Lektinzusammensetzung einen pH von 4 bis 9,5, vorzugsweise von 6 bis 8,5, vorzugsweise von 8,5 bis 9,5 aufweist.

In einer bevorzugten Ausführungsform ist die Zusammensetzung zur oralen Verabreichung bestimmt.

In einem weiteren Aspekt betrifft die Offenbarung eine Zusammensetzung wie oben beschrieben a. zur Verwendung zur Prophylaxe oder/und Behandlung von (AHT) Aromatase-Hemmer-, Antiöstrogen- oder/und GnRH-Analoga - bedingten unerwünschten Arzneimittelnebenwirkungen umfassend Austrocknen des gesamten Schleimhautkomplexes umfassend Augen-, Nasen-, Mund-, Rachen-, Vaginalschleimhäute sowie der des Magen-Darm-Traktes, Gelenkschmerzen, insbesondere morgendlicher Anlaufschmerz und Hitzewallungen oder b. zur Verwendung zur Prophylaxe oder/und Behandlung von Chemo- oder Strahlentherapie induzierten Arzneimittelnebenwirkungen umfassend Übelkeit, Erbrechen/Durchfall, trockene Schleimhäute, Gelenkbeschwerden, Müdigkeit/Schwäche, Mundtrockenheit oder c. zur Verwendung zur Prophylaxe oder/und Behandlung von natürlichem Hormondefizit bedingt durch die Wechseljahre, umfassend Hitzewallungen, Augentrockenheit, Trockenheit der Vaginalschleimhaut sowie Gelenkprobleme und einer erhöhten Rate von Infektionen im urogenitalen Bereich, Gewichtszunahme, übermäßige Nervosität sowie Empfindlichkeit, Müdigkeit, psychische Probleme wie Angstzustände, Schlafstörungen und depressive Zustände, oder d. zur Verwendung zur Prophylaxe oder/und Behandlung von durch Wachstumsrezeptorinhibitoren - bedingten , oder von durch Proteininhibitoren - bedingten, oder von durch Immuntherapeutika-bedingten unerwünschten Arzneimittelnebenwirkungen umfassend Austrocknen des gesamten Schleimhautkomplexes umfassend Augen-, Nasen-, Mund-, Rachen-, Vaginalschleimhäute sowie der des Magen-Darm-Traktes, Gelenkschmerzen, insbesondere morgendlicher Anlaufschmerz und Hitzewallungen, oder Übelkeit, Erbrechen/Durchfall, trockene Schleimhäute, Gelenkbeschwerden, Müdigkeit/Schwäche, Mundtrockenheit, oder Hitzewallungen, Augentrockenheit, Trockenheit der Vaginalschleimhaut sowie Gelenkprobleme und einer erhöhten Rate von Infektionen im urogenitalen Bereich, Gewichtszunahme, übermäßige Nervosität sowie Empfindlichkeit, Müdigkeit, psychische Probleme wie Angstzustände, Schlafstörungen und depressive Zustände.

In einem weiteren Aspekt betrifft die Offenbarung eine Zusammensetzung wie oben beschrieben, wobei der Wachstumsrezeptorinhibitor ausgewählt ist aus der Gruppe bestehend aus Intrazelluläre Tyrosinkinase-Inhibitoren und Extrazelluläre Rezeptor-Inhibitioren, der Proteininhibitor ausgewählt ist aus der Gruppe bestehend aus AKT Inhibitoren, ALK Inhibitoren, Angiogenese Inhibitoren, BCR-ABL Inhibitoren, B-Raf Inhibitoren, CDK Inhibitoren, Hedgehog Signaling Inhibitoren, IGF Signalweg Inhibitoren, cMET Inhibitoren, MEK Inhibitoren, mTOR Inhibitoren, PARP Inhibitoren, PI3K & PIK3CA Inhibitoren und (Tyrosin) Kinase Inhibitoren, oder das Immuntherapeutikum ausgewählt ist aus der Gruppe bestehend aus Immun-Checkpoint-Inhibitoren und Immunmodulatoren.

Insbesondere können die ausgewählten Substanzen eine oder mehrere aus der folgenden Liste sein:

Diethylstilbestrol, Fulvestrant, Tamoxifen, Toremifen, Abirateron, Anastrozol Exemestan, Letrozole, Bicalutamid, Enzalutamid, Flutamid, Abarelix, Degarelix, Goserelin, Leuprorelin, Triptorelin, Octreotid, Medroxyprogesterone, Megestrol, Afatinib (EGFR), Brigatinib (ALK, EGFR), Dacomitnib (EGFR) Erlotinib (EGFR,HER1R), Gefitinib (EGFR), Lapatinib (EGFR,HER2) Lenvatinib (VEGFR1,2,3, FGFR1,2,3,4, PDGFR alpha,c-Kit,RET) Neratinib (EGFR, HER2), Osimertinib (EGFR T790M), Sunitinib (PDGF,VEGF, c-Kit, FLT, CSF, RET) Vandetanib (VEGFR, EGFR,RET), Bevacizumab (VEGF), Cetuximab (EGFR), Dinutuximab (Gangliosid GD2), Necitumumab (EGFR), Olaratumab (PDGFR alpha), Panitumumab (EGFR), Pertuzumab (HER2), TDM1(Ado-trastuzumab emtansine) (HER2), Trastuzumab (HER2), Alectinib, Ceritinib, Crizotinib (ALK, MET, ROS1), Lorlatinib (ALK, ROS1), Aflibercept, Dasatinib (BCR-ABL, SRC), Imatinib (BCR-ABL), Nilotinib (BCR-ABL), Dabrafenib, Encorafenib, Vemurafenib, Abermaciclib, Palbociclib, Ribociclib, Sonidegib, Vismodegib, Cabozantinib, Binimetinib, Cobimetinib, Trametinib, Everolimus, Temsirolimus, Niraparib
Olaparib, Rucaparib, Talazoparib, Aspirin, Idelalisib, Nintedanib, Pazopanib (VEGFR, PDGFR, KIT), Regorafenib (VEGFR, TIE2, KIT, RET, RAF-1, BRAF, BRAFV600E, PDGFR, FGFR), Sorafenib (Raf,tyrosine kinases (VEGF)), Atezolizumab (PD-L1), Avelumab (PD-L1), Durvalumab (PD-L1), Ipilimumab (CTLA-4), Nivolumab (PD-1), Pembrolizumab (PD-1), Interleukin-2.

## Beispiele

Beispiel I.

Diese klinische Untersuchung beschreibt die Behandlung von unerwünschten Arzneimittelnebenwirkungen und Wechseljahresbeschwerden mit der Substanzkombination aus Enzymen, Selen in konstanter Dosierung und Linsenextrakt mit unterschiedlichen Lektin - Konzentrationen.

Mit Chemo/Strahlen oder Antihormontherapie behandelte Patienten mit wechseljahresähnlichen Symptomen wurden nach dem Zufallsprinzip in verschiedene Behandlungsgruppen eingeteilt.

Jede Gruppe erhielt parallel zu der Chemo/Strahlen oder Antihormontherapie täglich eine Behandlung mit den Bestandteilen Enzymen (400mg Papain und 400mg Bromelain), Na-Selenit (300μg) und Linsenextrakte in verschiedenen Lektinkonzentrationen wie folgt:

Gruppe I: Chemo/Strahlen oder Antihormontherapie ohne parallele Behandlung (Kontrollgruppe),

Gruppe II: 2 x täglich Enzyme 200 mg Papain und 200 mg Bromelain, Na-Selenit 150 μg und 10 mg Linsenextrakt (Tagesdosis: 20mg Linsenextrakt -Lektingehalt 1:1 parallel zu den Krebsstandarttherapien

Gruppe III: 2 x täglich Enzyme 200 mg Papain und 200 mg Bromelain, Na-Selenit 150 μg und 10 mg Linsenextrakt (Tagesdosis: 20mg Linsenextrakt Lektingehalt 1:100 parallel zu den Krebsstandarttherapien

Gruppe IV: 2 x täglich Enzyme 200 mg Papain und 200 mg Bromelain, Na-Selenit 150 μg und 10 mg Linsenextrakt (Tagesdosis: 20mg Linsenextrakt mit Lektingehalt 1:200 parallel zu den Krebsstandarttherapien

Gruppe V: 2 x täglich Enzyme 200 mg Papain und 200 mg Bromelain, Na-Selenit 150 μg und 10 mg Linsenextrakt (Tagesdosis: 20mg Linsenextrakt mit Lektingehalt 1:500 parallel zu den Krebsstandarttherapien

Vor der parallelen Zusatzbehandlung wurden die verschiedenen unerwünschten Arzneimittelnebenwirkungen bei den Patienten mittels einer Skala von 1 bis 6 (1: Nebenwirkung sehr schwach, 2: Nebenwirkung unangenehm, 3: Nebenwirkung sehr unangenehm, 4: Nebenwirkung stark, 5: Nebenwirkung sehr stark 6: Nebenwirkung unerträglich stark, ermittelt).

Nach einer Behandlungsdauer von 25 Tagen wurden erneut bei jedem Patienten die unerwünschten Nebenwirkung mittels der oben beschriebenen Skala bewertet und wie beschrieben in jeder Gruppe für jede der beurteilten Nebenwirkungen der Mittelwert errechnet. Gleichzeitig wurde die Verträglichkeit der adjuvanten Therapie nach dem Ende der Einnahme abgefragt(1=sehr gut vertragen; 2= gut vertragen; 4= nicht gut vertragen: 6= Einnahme abgebrochen).

Die klinische Untersuchung (siehe auch Figur 1) zeigt, dass eine Lektindosis von ca. 0,2 bis 0,04 mg Lektin einerseits zu einer Verminderung der Nebenwirkungen und der Verträglichkeit sowie der Compliance der Patienten führt. Andererseits konnte unerwarteter Weise bei Verwendung von Lektin in diesem Bereich eine deutliche Wirksamkeitssteigerung erzielt werden, die man so nicht erwarten konnte und die überraschend war.

Beispiel II.

Im folgenden werden die Versuche und Versuchsergebnisse von drei anerkannten *in vitro* Bluttests dargestellt, die die Ergebnisse aus Beispiel I. und die klinischen Ergebnisse bestätigen. Die im folgenden dargestellten und beschriebenen *in vitro* Tests sind anerkannt zur Bestimmung von Immunparametern und ergänzen nicht nur die Befunde der klinischen Untersuchungen, sondern sie bestätigen diese Ergebnisse und darüber hinaus zeigen sie, dass weitere Lektinverdünnungsstufen einer erfindungsgemäßen Zusammensetzung bis zu ca. 1:500 bis ca. 1:1000 (im Vergleich zu bekannter Dosis) weiterhin verbesserte Wirkung aufweisen bevor diese bei weiterer Verdünnung des Lektingehaltes dann abfällt.

1. Burst-Test

Der Burst-Test dient der quantitativen Bestimmung der sauerstoffabhängigen, intrazellulären Inaktivierung mikrobieller Erreger in Granulozyten und Monozyten. Opsonierte Bakterien dienen als partikuläres Stimulans, das chemotaktische Peptid N-formyl-MetLeuPhe (fMLP) als physiologisches, schwaches Stimulans. Heparinisiertes Vollblut wird mit bzw. ohne Stimulans inkubiert.

Durch die Stimulation der Zellen werden reaktive Sauerstoffradikale innerhalb des Phagosoms freigesetzt und dadurch die Bakterien zerstört. Die Sauerstoffradikale werden indirekt durch Oxidation des fluorogenen Substrates Dihydrorhodamin 123 zu dem Fluorochrom Rhodamin nachgewiesen. Bei der durchflusszytometrischen Analyse wird sowohl der prozentuale Anteil oxidierender Zellen, als auch die lysosomale Enzymaktivität, die der freigesetzten Menge an Rhodamin pro Zelle entspricht, gemessen.

Der Test ermöglicht die Bestimmung des prozentualen Anteils von

Verschiedene Substanzen wie z.B. opsonisierte Partikel (durch Immunglobulinebesetzte und markierte Fremdkörper), Phorbolester, Lekitine, Immunkomplexe,Kalzium-Ionophore, cis-ungesättigte Fettsäuren, hierbei insbesondere Arachidonsäurederivate etc. können über unterschiedliche Wirkungs-Mechanismen die polymorphkernigen Neutrophilen zur Produktion von Sauertoffradikalen anregen(Baggiolini et al. 1993). In-vivo kommt es z.B. auch durch den Kontakt mit Bakterienoberflächen zum "oxidative burst" der PMN, d.h. innerhalb kürzester Zeit zur massiven Erzeugung von reaktionsfähigen Sauerstoffprodukten. Im in-vitro Experiment kann man sich entsprechend des Oligopeptids N-formyl Met-Leu-Phe (fMLP) bedienen, um eine Aktivierung der Neutrophilen zu erreichen. fMLP ist ein aus drei Aminosäuren bestehendes Peptid und wirkt über einen spezifischen fMLP-Rezeptorauf der PMN-Zelloberfläche (Bengis-Garber et al. 1991). Dem Rezeptor ist ein G-Protein nachgeschaltet, das zur Aktivierung der Phospholipase C (PLC) überleitet (Smolen 1992). fMLP ist in der Lage, alle drei Phospholipasen (PLA2, PLCund PLD) zu aktivieren (Cockcroft et al.1992). Über spezielle "sonder-messenger"-Wege kommt es schließlich zur Aktivierung der Proteinkinase C, der über einen nochunklaren Mechanismus die Aktivierung der NADPH-Oxidase folgt.

fMLP bewirkt eine Erhöhung der intrazellulären Kalziumkonzentration, dabei wird einerseits Kalzium von intrazellulären Kompartimenten mobilisiert andererseits der extrazelluläre Ca++-Influx gesteigert. Die PLC und auch die NADPH-Oxidase sind kalziumabhängige Enzyme. Der Aktivierung der NADPH-Oxidase geht ein Aggregation des aus mehreren Teilen bestehenden Enzymkomplexes voraus.

Durch das starke Stimulans wird ein maximaler oxidativer Burst in den Leukozyten hervorgerufen ("high control"), während durch das schwache Stimulans nur eine vergleichsweise schwache Reaktion ausgelöst wird ("low control"), bei der man auch additive Effekte von weiteren Prüfsubstanzen, z.B. Immunmodulatoren (Tumor-Nekrosis-Faktor (TNF), Granulozyten-Makrophagen-Koloniestimulierender Faktor (GM-CSF)) bestimmen kann.

Auszüge aus der Standard Operational Procedure (SOP)

Probenmaterial

Frisches Heparin-Blut (10 ml max. 24 Stunden alt, ungeronnen)

Probenvorbereitung

- D N A -Solution, Waschlösung auf Eis stellen
- Substrat 1 : 10 mit Waschlösung verdünnen (erst Substrat und dann Waschlösung pipettieren)
- Spitzröhrchen mit AP-Nr. beschriften und auf Eis stellen (mit Parafilm verschließen)
- FMLP 1 : 101 mit Waschlösung verdünnen (z. B. 10 und 1000 µl)
- Portionierte E.coli aus dem - 80 °C Gefrierschrank holen
- Ca. 1 ml Heparinblut vorher gut schwenken in Glasröhrchen geben und für 15 Min. auf Eis stellen (Monozyten lösen sich ab)
- Je 100 µl gekühltes Heparinblut in Spitzröhrchen verteilen
- In das Kontroll-Röhrchen werden 20 µl Waschlösung pipettiert
- In das FMLP-Röhrchen werden 20 µl verdünnte FMLP-Lösung pipettiert
- I In das FMLP-Röhrchen werden 20 µl verdünnte FMLP-Lösung + Testsubstanz pipettiert
- in das E. coli-Röhrchen werden 20 µl E. coli () pipettiert
- Mischen und 10 Min. bei 37 °C im Wasserbad inkubieren (alle 3 Röhrchen)
- 20 µl Substrat dazupipettieren, mischen und 10 Min. bei 37 °C inkubieren (alle 3 Röhr.)
- 2 ml Lyse dazupipettieren
- 15 Min. bei RT inkubieren
- danach 5 Min. bei 250 g bei 4 °C zentrifugieren
- Überstand mit Pasteurpipette absaugen
- 3 ml Waschlösung dazugeben und mischen
- danach 5 Min. bei 250 g 4 °C zentrifugieren
- Überstand mit Pasteurpipette absaugen
- 100 µl D N A-Solution pipettieren und mischen
- 10 Min. auf Eis inkubieren und innerhalb 30 Min. messen

Bestimmung des oxidativen Burst:
Messung am Durchflusszytometer

2. LTT-Test

Der Lymphozyten Funktionstest (LTT-Test) ist der wichtigste Funktionstest, der über den Aktivierungsgrad der Abwehrzellen Auskunft gibt und verschiedene Immunstimulantien an patienteneigenen Lymphozyten auf deren Wirkungsgrad testet. Mit dem LTT kann zusätzlich zur aktuellen Immunkompetenz das individuelle Ansprechen verschiedener immunstimulierender Präparate objektiviert werden.

Auszüge aus der Standard Operational Procedure (SOP)

Probenmaterial

20 - 30 ml frisches Natrium-Heparin-Blut, bis ca. 24 h nach Abnahme (Lagerung und Transport bei 10 °C - 37 °C) oder 2 - 3 CPT-Röhrchen (Fa. Becton Dickinson) gefugt, bis 48 h nach Abnahme (Lagerung und Transport bei 15 °C - 40 °C) und wenn möglich 1 Vollblut für autologes Patientenserum. Durchführung der Analyse innerhalb von 24 h. Hämolytische Proben (Schädigung durch den Transport) oder Blutproben die definitiv zu alt sind (Transport über das Wochenende oder Feiertage) oder geronnene Proben können nicht weiterverarbeitet werden (Textbaustein-Liste = Formblatt 6) - falls solche Proben in Ausnahmefällen doch angesetzt werden müssen, wird dies auf dem Sonderbefund vermerkt.

Allgemein/ Testprinzip

Die mononukleäre Zellfraktion des peripheren Blutes enthält auch sog. Gedächtniszellen, die im Rahmen einer Primärreaktion (bei Kontakt mit einem spez. Antigen) gebildet werden. Bei Rekontakt mit dem spezifischen Antigen werden diese Memory-Zellen aktiviert und proliferieren. Die damit einhergehende DNS-Neusynthese kann in vitro über den Einbau der BrdU quantifiziert werden. In der Regel werden spezifische Memory-T-Lymphozyten über MHC II-Präsentation des prozessierten Antigens auf Antigenpräsentierenden Zellen aktiviert. Die Methode kann sowohl für die Überprüfung der Funktion des zellulären Immunsystems als auch zum Infektionsnachweis oder zum Nachweis von Spättypsensibilisierungen gegenüber Allergenen eingesetzt werden. Weiterhin kann mit dieser Methode eine erhöhte Prävalenz an autoreaktiven T-Zellen im peripheren Blut nachgewiesen werden.

Herstellung der Antigenpräparationen

Die Herstellung der Antigenpräparationen erfolgt unter Sterilbedingungen mit steriler, endotoxinfreier PBS-Lösung (Gibco). Das Abwiegen der Reinsubstanzen erfolgt auf der Analysenwaage. Für die einzelnen Substanzgruppen gelten folgende Besonderheiten:

- Proteine/Lyophilisate: werden in PBS gelöst und in Aliquots bei -65°C bis -90°C eingefroren.

Belegung der Mikrotiterplatten

- In der Mikrotiterplatte werden pro Well 20 µl der Antigenlösung vorgelegt

  10 µl PWM (Achtung: LTTA 20 µl PWM)
  20 µl PBS (Basalwert)

- Die Belegung der Mikrotiterplatten mit den Testlösungen für die einzelnen Profile ist aus Formblatt 1 (Plattenbelegung) ersichtlich.
- Für alle Testansätze müssen die Mikrotiterplatten frisch angesetzt werden.
- Die Mikrotiterplatten sind mit dem entsprechenden Profil, der Auftragsnummer und dem Ansatzdatum zu beschriften.

Kontrollen

Leerwert: Basalwert = 200.000 Zellen (entsprechend 200 µl Zellsuspension) unter Zugabe von 20 µl PBS

Plausibilitätskontrolle: PWM-Mitogen = 200.000 Zellen (entsprechend 200 µl Zellsuspension) unter Zugabe von 10 µl PWM

Aufbereitung der mononukleären Zellfraktion

- 15ml Ficoll in 50ml Rörchen vorlegen und vorsichtig mit Blut überschichten und mit PBS auf 50 ml auffüllen. 18 min. bei 800 x g und Raumtemperatur zentrifugieren. CPT-Röhrchen (falls unzentrifugiert) werden 18 min. bei 1600 x g zentrifugiert
- Nach Beendigung der Zentrifugation wird der weiße Lymphozytenring mit einer sterilen Transferpipette in eine beschriftete (Auftragsnummer und Profil), sterile 50 ml Schraubröhre überführt. Dabei ist zu beachten, daß keine Erythrozyten o. ä. mit abgesaugt werden.
- Die Lymphozyten werden gewaschen:

  - Die Zellsuspension wird mit PBS auf 50 ml aufgefüllt

  - Röhrchen 10 min. bei 250 x g zentrifugieren und anschließend den Überstand verwerfen

  - das Pellet mit 1 ml PBS resuspendieren und auf 10 ml auffüllen (PBS)

    - Waschschritt bis zum Verwerfen des Überstandes wiederholen

- Vorbereitung des Kulturmediums:

  In 500 ml Medium (RT) werden Zusätze (Herstellung und Haltbarkeit lt. Formblatt 3) gegeben und gemischt (2 Tage im Kühlschrank haltbar).

  100 ml des Mediums (+ Zusätze) wird mit 5 µl Interferon-α (125 U/ml) versetzt - ACHTUNG nur 2 Std. haltbar

- Nachdem der Überstand verworfen wurde, wird das Pellet mit 1 ml des frisch hergestellten Kulturmediums (+ Interferon-α) resuspendiert und diese Zellsuspension mit Kulturmedium auf 10 ml aufgefüllt.
- Achtung: LTTA, LTTAK, LTTIM und LTTBOR wird das Pellet ohne Interferon-α resuspendiert und auf 10 ml aufgefüllt

Ermittlung der Zellzahl und Probenansatz:

Cell Proliferation ELISA BrdU (colorimetric) der Firma Roche

Elisa Durchführung

- Alle Reagenzien auf Raumtemperatur bringen
- Jedes Well mit 20 µl BrdU labeling solution (= BrdU labeling reagent 1:100 mit sterilem RPMI-Medium verdünnt) markieren
- 16 - 18 Stunden im CO2-Brutschrank (unter gleichen Bedingungen wie unter Punkt 4.11.2 beschrieben) inkubieren
- 10 Minuten bei 300xg zentrifugieren
- 1 Stunde bei 60 °C trocknen (oder 15 Minuten föhnen bzw. Geltrockner)
- 200 µl FixDenat (gebrauchsfertig) in jedes Well pipettieren
- 30 Minuten bei Raumtemperatur inkubieren
- Platte abkippen und auf Saugpapier trocken klopfen
- 100 µl anti-BrdU-POD working solution (= anti-BrdU-POD stock solution 1:100 mit antibody dilution solution verdünnt) in jedes Well pipettieren - Achtung: immer kurz vor Gebrauch ansetzen
    - anti-BrdU-POD stock solution (= Fläschchen 3): vor dem ersten Gebrauch mit 1,1 ml dd$H_2O$ auflösen und 10 Minuten mischen
- 90 Minuten bei Raumtemperatur inkubieren
- Platte abkippen und auf Saugpapier trocken klopfen
- 3 mal mit 200 µl washing solution (= wasching buffer 1:10 mit d$H_2O$ verdünnt) waschen
- 100 µl Substrat (gebrauchsfertig) in jedes Well pipettieren
- 5 Minuten bei Raumtemperatur inkubieren
- 25 µl stop solution (= 1M $H_2SO_4$ nicht im Kit enthalten) in jedes Well pipettieren
- innerhalb von 5 Minuten nach Abstoppen der Reaktion bei 450 nm im Plattenphotometer messen

**3. NK-Zellfunktionstest /Modulator**

Mit dieser Standardarbeitsanweisung wird die durchflusszytometrische NK-Zytotoxizitätstest-Bestimmung unter Einfluss bestimmter Medikamente festgelegt.

Testprinzip / Reaktionsprinzip der Methode

Inkubation von Patientenzellen mit Immunmodulatoren, anschließend Messung der zytotoxischen Aktivität gegenüber K 562 Tumorzellen. Feststellung des optimalen Immunmodulator zur in-vivo Anwendung.

Probenmaterial

Heparinblut-Blut (ca. 20 ml, maximal 24 h alt) bei RT, nicht geronnen Probenvorbereitung

Material: möglichst 2 NH-Röhrchen!

- Ficolltrennung (siehe Formblätter SOP NK-Zellplatz)
- Patientenzellen auszählen (siehe SOP-MU-IMM.M.0009.xx) und auf 1 x $10^6$/ml einstellen:
- Verdünnungsreihe der benötigten Medikamente herstellen:
- Stimulationsansatz in Facs-Röhrchen

| | |
|---|---|
| Basal: | 900 µl Medium + 100 µl Patientenzellsuspension |
| IL2 positive Kontrolle: | 900 µl Medium + 100 µl Patientenzellsuspension + 10µl IL2 |

Medikament (jede der 3 Verdünnungen):

800 µl Medium + 100 µl Patientenzellsuspension + 100 µl Medikamentenverdünnung

- Röhrchen über Nacht bei 37 Grad + 5 % CO2 im Brutschrank (IMMUTOX) inkubieren
- Ansatz für (%) NK-Zellanteilbestimmung:
  - 10 µl Antikörper (CD3/ CD16+CD56) in ein Facs-Röhrchen vorlegen
  - 100 µl Patientenzellsuspension zugeben
  - 15 Min. bei RT inkubieren
  - 100 µl Flow Count Beads (am Morgen gut vortexen; vor Gebrauch nochmals kurz gut mischen) + ca. 1 ml PBS zugeben
  - am FC 500, unter Protokoll (Resource Explorer) NK-Zellen Flow count messen.
  - % Angabe der NK-Zellen wird Region C1 entnommen.
  - Ausdruck am Arbeitsplatz aufbewahren und am nächsten Tag mit den restlichen Messungen zusammenheften.
- Über Nacht kultivierte Zellsuspensionen (1 ml) 10 Min. bei 800 UpM zentrifugieren und abgießen.
- Gefärbte K 562 Tumorzellen fugen (10 min., 500UpM), abgießen und mit 1 ml K562-Medium auffüllen und auf 1 x 10^5 einstellen (siehe Beilagen SOP NK-Zellplatz)
- Berechnung benötigter K562-Zellmenge (im Verhältnis 1:1 mit den absoluten NK-Zellen des Patienten). Absolut-Wert wird der Spalte Count beim % NK-Zellanteil entnommen z.B. C1= 5% und Count= 115
- Zum Patienten-Zellpellet K 562 Tumorzellen hinzu pipettieren (siehe Berechnung benötigter K562-Zellmenge) z.B. 115µl (aber mindestens 100µl bis max. 300µl)
- Ein Röhrchen mit mind. 200µl K562 Tumorzellen und mind. 200µl Medium mitführen (K 562 Kontrolle)
- Alle Röhrchen 3 - 4 Min. bei ca. 500 UpM zentrifugieren (ohne Bremse)
- Röhrchen mit Aluminiumfolie abdecken und 2 Stunden bei 37°C im Brutschrank inkubieren danach auf Eis stellen.
- 100 µl DNA - Färbelösung (Propidiumjodidlösung) zum Anfärben der DNA in jedes Röhrchen pipettieren und vortexen 10 Min. im Dunkeln auf Eis stehen lassen und innerhalb von 30 Min. am FC 500 messen

Messung am Durchflusszytometer

Referenzbereich

$$\text{Basal} > 25\%$$

$$\text{IL2} > 40\%$$

$$\text{Medikamente (Modulatoren)} > 25\%$$

NK-Zellaktivierung unter Einfluss bestimmter Modulatoren

Testprinzip / Reaktionsprinzip der Methode

Inkubation von Patientenzellen mit Immunmodulatoren, anschließend Messung der zytotoxischen Aktivität mit CD69. Feststellung des optimalen Immunmodulator zur in-vivo Anwendung.

Probenmaterial

Heparinblut-Blut (ca. 20 ml, maximal 24 h alt) bei RT, nicht geronnen
Probenvorbereitung
Material: möglichst 2 NH-Röhrchen!
• Ficolltrennung (siehe Formblätter SOP NK-Zellplatz)
• Patientenzellen auszählen (siehe SOP-MU-IMM.M.0009.xx) und auf 1 x $10^6$/ml einstellen:
• Verdünnungsreihe der benötigten Medikamente herstellen:
Medikamente auf 1 mg/ml einstellen (falls keine Konzentrationsangaben vorhanden, Verdünnung 1:10 oder 1:50, so dass keine Verfärbung beim Pipettieren in die 1. Verd.-Stufe erfolgt)
• Stimulationsansatz in Facs-Röhrchen

| | |
|---|---|
| Basal: | 900 µl Medium + 100 µl Patientenzellsuspension |
| IL2 positive Kontrolle: | 900 µl Medium + 100 µl Patientenzellsuspension + 10µl IL2 |

Medikament (jede der 3 Verdünnungen):
800 µl Medium + 100 µl Patientenzellsuspension + 100 µl Medikamentenverdünnung
• Röhrchen über Nacht bei 37 Grad + 5 % CO2 im Brutschrank inkubieren
• Über Nacht kultivierte Zellsuspensionen 10 Min. bei 800 UpM zentrifugieren und abgießen.
• Danach 10 µl CD3/CD16+56 und 5 µl CD69-PC7 in die FACS-Röhrchen pipettieren und 10 Minuten im Dunkeln inkubieren.
• Nach der Inkubation PBS zum Messen in die Röhrchen pipettieren und am Navios-DFZ messen (Panel: Medikament-(1,2).pnl)

Referenzbereich

Basal <15%

IL2 > 70%

Medikamente (Modulatoren) > 20%

**Zusammenfassung der klinischen und *in vitro* Tests**

Die oben beschriebenen drei *in vitro* Tests belegen die klinischen Ergebnisse aus Beispiel I.

Es konnte überraschender Weise gefunden werden, dass ein Lektingehalt bzw. eine Dosis an Lektin von ca. 0,1 bis 0,03 mg nicht nur zu einer Verminderung der Nebenwirkungen führt, sondern dass in diesem Lektinbereich überraschender Weise eine Wirksamkeitssteigerung erzielt werden kann.

Ein erfindungsgemäßer Bereich des Lektingehaltes von 0,1 bis 0,03 mg Lektin, vorzugsweise von 0,2 bis 0,04 mg Lektin, kann somit auch als optimaler Wirkbereich der Lektindosis bezeichnet werden.

Es war nicht zu erwarten gewesen und damit überraschend, dass man bei Verminderung der Lektindosis die Nebenwirkungen vermindern kann und gleichzeitig eine Wirkungssteigerung erzielen könnte. Damit stellt die Erfindung vorteilhafter Weise eine Lektinzusammensetzung zur Verfügung, die nicht nur den Vorteil einer Nebenwirkungsverminderung und damit besserer Compliance der Pateinten erreichen kann, sondern eine Lektinzusammensetzung, die vorteilhafter Weise eine verbesserte Wirkung zeigt.

Literatur

Literatur zu Burst-Test

(1) Roitt, I.M., Brostoff, J. & D.K. Male. 1996. Immunology, 4th ed. Gower Medical Publishing Ltd., London.

(2) Sawyer, D.W., Donowitz, G.R. & G.L. Mandell. 1989. Polymorphonuclear neutrophils: An effective antimicrobial force. Rev. Infect. Dis. 11: S1532-S1544.

(3) Donadebian, H. D. 1989. Congenital and acquired neutrophil abnormalities. In: Klempner, M.S. et al.(eds) Phagocytes and Disease. Kluwer, Dordrecht Boston New York, pp 103-118.

(4) Smith, R.M. & J.T. Curnutte. 1991. Molecular basis of chronic granulomatous disease. Blood 77: 673 -686.

(5) Rothe G, Oser A & G. Valet. 1988. Dihydrorhodamin 123: a new flow cytometric indicator for respiratory burst activity in neutrophil granulocytes. Naturwissenschaften 75: 354 - 355.

(6) Miyagawa, B. & H.-G. Klingemann. 1997. Phagocytosis and burst activity of granulocytes and monocytes after stem cell transplantation. J. Lab. Clin. Med. 129(6): 634-7.

(7) Dobmeyer, T.S., Raffel, B. Dobmeyer, J.M., Findhammer, S., Klein, S.A., Kabelitz, D. Hoelzer, D., Helm,E.B. & Rossol.1995. Decreased function of monocytes and granulocytes during HIV-1 infection correlates with CD4 cell counts. Eur. J. Med. Res. 1: 9-15. BURSTTEST Instructions, page 704/02 1002-05

8) Esparza, B., Sánchez, H., Ruiz, M., Barranquero, M., Sabino, E. & F. Merino. 1996. Neutrophil function inelderly persons assessed by flow cytometry. Immunol. Invest. 25(3): 185-190.

(9) Gessler, P., Nebe, T. Birle, A., Haas, N. & W. Kachel. 1996. Neutrophil respiratory burst in term and preterm neonates without signs of infection and in those with increased levels of C-Reactive Protein.Pediatr. Res. 39: 843-848.

(10) Elbim, C., Chollet-Martin, S., Bailly, S., Hakim, J. & M.A. Gougerot-Pocidalo. 1993. Priming of polymorphonuclear neutrophils by tumor necrosis factor in whole blood: Identification of two polymorphonuclear neutrophil subpopulations in response to formyl-peptides. Blood 82: 663-640.

Literatur zu NK-Zelltestung

Verbesserte Methode zur Untersuchung des Einflusses von Immunmodulatoren auf die zytotoxische Aktivität natürlicher Killerzellen

Improved Method for Testing the Influence of Immunmodulators on the Cytotoxic Activity of Natural Killer Cell

Monika-Gabriele Penz, A. Müller, S. Hofstetter and W. P. Bieger Laboratoriums Medizin Volume 26, Issue 5-6, pages 347-353, June 2002

Reichel M. et al; Pilotstudie zum Einfluß von NeyTumorin®-Sol auf Immunparameter von Karzinompatienten, Naturheilpraxis 5, 2-4 1998

Kadish A.S et al, Natural cytotoxicity and interferon production in human cancer... J.Immunol 127: 1817

Baxevanis C. et al; Mistletoe lectin I induced effects on human cytotoxic lymphocytes, Immunopharmacology and Immunotoxikology 20(3), 355-371 (1998)

Penz M; Aktuelle Diagnostikverfahren für eine individuell optimierte Tumortherapie mit komplementären Immuntherapeutikum Forum Komplementäre Onkologie & Forum Immunologie 1,14-18; 2000

Hülsen H et al; Einfluß von Mistelpräparaten auf die in vitro Aktivität der natürlichen Killerzellen von Krebspatienten Therapeutikum 7 (1993) 434

Schaaf J. et al.; Die natürliche Killerzellenaktivität als wichtiger Parameter bei Immuntherapien mit Biomodulatoren, Z. Onkol.28 (1996)

Whiteside R. et al. The role of natural killer cells in immune of cancer Current Biol. LTD 0952-7915

Mechelke F. Einfluß von Mistelpräparaten auf die Aktivität der natürlichen Killerzellen im peripheren Blut Uni Hohenheim

Riondel J et al. In vitro comparative study of cytolysis mediated by natural killer cells towards malignant cells preincubated with antioxidants, Anticancer research 18: 1757-1764 (1998)

Erpenbach K; Neue Erkenntnisse und Ergebnisse für eine intelligente und individuelle Therapie

Der Kassenarzt April 1998 18

Imai K et al. Natural cytotoxic activity of peripheral blood lymphocytes and cancer incidence

Lancet 356,25 1795-1799, 2000

Kim C.J. et al Antitumor Killer Lymphocytes in the peripheral blood of a patient with transitional cell carcinoma of the bladder; Int. J. Urol 1998; 5:230-236

Pross H.F. et al, Role of natural killer cells in cancer, Nat. Immun 1993,12:279

Schantz S.T et al Quantitation of natural killer cell function and risk of metastatic poorly differentiated head and neck cancer; Nat .Immun Cell growth Regul, 1991, 10: 278-288

Chakraborty A, et al, Age related natural killer activity periperal blood lymphocytes from healthy subjects and cancer patients, Tumori 1994,80(3): 233-237

Konjevic G; Evaluation of different effects of sera of breast cancer patients on the activity of natural killer cells, J-Clin. Lab. Immunol. 1992; 38(2):83-93

Ono K, Clinical significance of natural killing activity in patients with advanced lymphoma, J. of Clin. Immunol, 1998, 18:: 132-141

Shevde L.A. Natural killer cell function and genetic instability in unaffected individuals from breast cancer families, Europ. J. of Cancer prevention 1998, 7:141-148

Schnell R, Poyraz S, Radzuhn A, Hartwig MT, Drillich S, Schön G, Manzke O, Bohlen H, Diehl V, Engert A. Immune response and NK-cell function of Hodgkin's patients treated with an anti-CD25 immunotoxin (RFT5-SMPT-dgA). Onkologie, Suppl 2:
J. Wright, A. Young. Lorus Therapeutics, Toronto, Canada; D. Braun, Ohio Cancer Center, Toledo, Ohio. Stimulation of Natural Killer (NK) Cell Function in Pancreatic Cancer with Virulizin (V), an Immunotherapeutic Agent.

Tabelle A (zu Figur 1)

Beschwerdereduktion bei Prüfpräparat mit unterschiedlichen Lektingehalten

| | Ausgangswerte | Lektingehalt 1:1 | Lektingehalt 1:100 | Lektingehalt 1:200 | Lektingehalt 1:500 |
|---|---|---|---|---|---|
| Gelenkbeschwerden | 4,8 | 2,5 | 1,8 | 1,9 | 2,5 |
| Morgendliche Anlaufschmerzen | 4,5 | 2,3 | 1,7 | 1,9 | 2,2 |
| Trockene Schleimhäute | 4,2 | 3 | 1,9 | 2 | 2,7 |
| Mundtrockenheit | 3,9 | 2,6 | 1,6 | 1,8 | 2,2 |
| Augenbrennen | 3,6 | 2,7 | 1,5 | 1,8 | 2,6 |
| Vaginaltrockenheit | 4,3 | 2,8 | 1,6 | 1,9 | 2,8 |
| Trockene Haut/Hautreizung | 3,5 | 3 | 2,1 | 2,1 | 2,7 |
| Müdigkeit/Schwäche | 4 | 3,2 | 2,7 | 2,8 | 3 |
| Mastopathien | 3 | 2,2 | 1,6 | 1,9 | 2,2 |
| Dünnerwerden der Haare | 1,2 | 3,4 | 2,8 | 2,5 | 3 |

Tabelle B (zu Figur 5)

Verträglichkeit der begleitenden Therapie

| | Gruppe I: Ohne parallele Behandlungskontrolle (n = 18) | Gruppe II: 2 x 1 Tabletten Enzyme Selen Linsenextrakt Linsenlektin (1 : 1) 20 mg/die (n = 12) | Gruppe III: 2 x 1 Tabletten Enzyme Selen Linsenextrakt Linsenlektin (1 : 100) 0,2 mg/die | Gruppe IV: 2 x 1 Tabletten Enzyme Selen Linsenextrakt Linsenlektin (1 : 200) 0,1 mg/die | Gruppe V: 2 x 1 Tabletten Enzyme Selen Linsenextrakt Linsenlektin (1 : 500) 0,04 mg/die |
|---|---|---|---|---|---|
| 1 Habe die Begleitmedikation sehr gut vertragen | - - | 1 = 8 % | 4 = 25 % | 4 = 29 % | 5 = 36 % |
| 2 Habe die Begleitmedikation gut vertragen | - - | 5 = 43 % | 10 = 63 % | 9 = 64 % | 9 = 64 % |
| 4 Habe die Begleitmedikation nicht gut vertragen | - - | 4 = 31 % | 2 = 12 % | 1 = 7 % | 0 |
| 6 Habe die Begleitmedikation abgesetzt | - - | 2 = 18 % | 0 | 0 | 0 |
| Durchschnitt | - - | 3,25 | 2,7 | 1,8 | 1,6 |

## Patentansprüche

1. Zusammensetzung umfassend mindestens einen Lektin-haltigen Linsenextrakt, wobei der Gehalt an Linsenlektin (Lens culinaris) 0,5 bis 0,08 mg beträgt und gegebenenfalls weitere Zusatz- und Hilfsstoffe und wobei die Zusammensetzung eine pharmazeutische Zusammensetzung, ein diätetisches Lebensmittel, ein Lebensmittel für die bilanzierte Diät, ein Nahrungsergänzungsmittel oder ein Medizinprodukt ist.

2. Zusammensetzung nach Anspruch 1, wobei die Zusammensetzung weiterhin umfasst mindestens eine Selenverbindung und/oder mindestens ein proteolytisches Enzym oder/und Vitamin D oder/und Vitamin B2 oder/und Zink oder/und Curcumin.

3. Zusammensetzung nach einem der vorhergehenden Ansprüche, wobei das Selen ein Natrium-Selenit, ein Kalium-Selenit, oder ein Magnesium-Selenit ist, wobei vorzugsweise die Tagesdosis der Selenverbindung mindestens 25-500 $\mu$g, vorzugsweise 50-300 $\mu$g, der Selenverbindung beträgt.

4. Zusammensetzung nach einem der vorhergehenden Ansprüche, wobei das proteolytische Enzym ein Papain, ein Bromelain, ein Trypsin oder/und ein Chymotrypsin ist, und wobei vorzugsweise die Tagesdosis der proteolytischen Enzyme mindestens 10-1000 mg oder 50-5000 FIP-Einheiten, vorzugsweise 20 - 1000 mg oder 100-5000 FIP-Einheiten, beträgt.

5. Zusammensetzung nach einem der vorhergehenden Ansprüche, wobei die weiteren Zusatz- und Hilfsstoffe ausgewählt sind aus der Gruppe bestehend aus Phospholipiden, vorzugsweise Phosphoglyceride, Mizellstabilisatoren, vorzugsweise ausgewählt aus der Gruppe bestehend aus Carnitin oder/und einem physiologisch verträglichen Carnitin-Derivat oder/und Polyol(en), oder/und Radikalfängern, vorzugsweise ausgewählt aus der Gruppe bestehend aus Ascorbinsäure oder/und Alkali- oder/und Erdalkalisalze der Ascorbinsäure oder/und Ester einer oder mehrerer organischer Säuren mit Ascorbinsäure.

**6.** Zusammensetzung nach einem der vorhergehenden Ansprüche, wobei die Zusammensetzung zubereitet ist als eine Tablette, eine Kautablette, eine Retardtablette, eine Kapsel, ein Dragee, ein Pulver, ein Granulat, ein Lyophilisat, eine Suspension oder als wässrige oder ölige Lösung, Emulsion, Nanoemulsion, Nanodispersionssystem.

**7.** Zusammensetzung nach einem der vorhergehenden Ansprüche, wobei das Lektin emulgiert vorliegt in Vesikeln, Mischmizellen, als Mikroemulsion, als nanodisperses System oder Nanoemulsion, oder als flüssige, wässrige, mizellare Emulsion oder als flüssige, wässrig-ölige, mizellare Emulsion.

**8.** Zusammensetzung nach Anspruch 7, wobei die Mizellen einen mittleren Durchmesser von 3 bis 300nm, vorzugsweise von 5 bis 250nm, besonders bevorzugt von 10 bis 120nm, mehr bevorzugt von 50 bis 80nm aufweisen.

**9.** Zusammensetzung nach Anspruch 7 oder 8, wobei die Emulsion der Lektinzusammensetzung einen pH von 4 bis 9,5, vorzugsweise von 6 bis 8,5, vorzugsweise von 8,5 bis 9,5 aufweist.

**10.** Zusammensetzung nach einem der vorhergehenden Ansprüche, wobei die Zusammensetzung zur oralen Verabreichung bestimmt ist.

**11.** Zusammensetzung nach einem der vorhergehenden Ansprüche a. zur Verwendung zur Prophylaxe oder/und Behandlung von (AHT) Aromatase-Hemmer-, Antiöstrogen- oder/und GnRH-Analoga - bedingten unerwünschten Arzneimittelnebenwirkungen umfassend Austrocknen des gesamten Schleimhautkomplexes umfassend Augen-, Nasen-, Mund-, Rachen-, Vaginalschleimhäute sowie der des Magen-Darm-Traktes, Gelenkschmerzen, insbesondere morgendlicher Anlaufschmerz und Hitzewallungen oder b. zur Verwendung zur Prophylaxe oder/und Behandlung von Chemo- oder Strahlentherapie induzierten Arzneimittelnebenwirkungen umfassend Übelkeit, Erbrechen/Durchfall, trockene Schleimhäute, Gelenkbeschwerden, Müdigkeit/Schwäche, Mundtrockenheit oder c. zur Verwendung zur Prophylaxe oder/und Behandlung von natürlichem Hormondefizit bedingt durch die Wechseljahre, umfassend Hitzewallungen, Augentrockenheit, Trockenheit der Vaginalschleimhaut sowie Gelenkprobleme und einer erhöhten Rate von Infektionen im urogenitalen Bereich, Gewichtszunahme, übermäßige Nervosität sowie Empfindlichkeit, Müdigkeit, psychische Probleme wie Angstzustände, Schlafstörungen und depressive Zustände, oder d. zur Verwendung zur Prophylaxe oder/und Behandlung von durch Wachstumsrezeptorinhibitoren - bedingten , oder von durch Proteininhibitoren - bedingten, oder von durch Immuntherapeutika-bedingten unerwünschten Arzneimittelnebenwirkungen umfassend Austrocknen des gesamten Schleimhautkomplexes umfassend Augen-, Nasen-, Mund-, Rachen-, Vaginalschleimhäute sowie der des Magen-Darm-Traktes, Gelenkschmerzen, insbesondere morgendlicher Anlaufschmerz und Hitzewallungen, oder Übelkeit, Erbrechen/Durchfall, trockene Schleimhäute, Gelenkbeschwerden, Müdigkeit/Schwäche, Mundtrockenheit, oder Hitzewallungen, Augentrockenheit, Trockenheit der Vaginalschleimhaut sowie Gelenkprobleme und einer erhöhten Rate von Infektionen im urogenitalen Bereich, Gewichtszunahme, übermäßige Nervosität sowie Empfindlichkeit, Müdigkeit, psychische Probleme wie Angstzustände, Schlafstörungen und depressive Zustände.

**12.** Zusammensetzung zur Verwendung nach Anspruch 11, wobei der Wachstumsrezeptorinhibitor ausgewählt ist aus der Gruppe bestehend aus Intrazelluläre Tyrosinkinase-Inhibitoren und Extrazelluläre Rezeptor-Inhibitioren, der Proteininhibitor ausgewählt ist aus der Gruppe bestehend aus AKT Inhibitoren, ALK Inhibitoren, Angiogenese Inhibitoren, BCR-ABL Inhibitoren, B-Raf Inhibitoren, CDK Inhibitoren, Hedgehog Signaling Inhibitoren, IGF Signalweg Inhibitoren, cMET Inhibitoren, MEK Inhibitoren, mTOR Inhibitoren, PARP Inhibitoren, PI3K & PIK3CA Inhibitoren und (Tyrosin) Kinase Inhibitoren, oder das Immuntherapeutikum ausgewählt ist aus der Gruppe bestehend aus Immun-Checkpoint-Inhibitoren und Immunmodulatoren.

## Claims

**1.** A composition comprising at least one lectin-containing lentil extract, wherein the content of lentil lectin (lens culinaris) is 0.5 to 0.08 mg and optionally further additives and adjuvants and wherein the composition is a pharmaceutical composition, a dietetic food, a food for the balanced diet, a food supplement or a medical product.

2. The composition according to claim 1, wherein the composition further comprises at least one selenium compound and/or at least one proteolytic enzyme or/and vitamin D or/and vitamin B2 or/and zinc or/and curcumin.

3. The composition according to any one of the preceding claims, wherein the selenium is a sodium selenite, a potassium selenite, or a magnesium selenite, wherein preferably the daily dose of the selenium compound is at least 25-500 μg, preferably 50-300 μg, of the selenium compound.

4. The composition according to any one of the preceding claims, wherein the proteolytic enzyme is a papain, a bromelain, a trypsin or/and a chymotrypsin, and wherein preferably the daily dose of the proteolytic enzymes is at least 10-1000 mg or 50-5000 FIP units, preferably 20-1000 mg or 100-5000 FIP units.

5. The composition according to any one of the preceding claims, wherein the further additives and auxiliary substances are selected from the group consisting of phospholipids, preferably phosphoglycerides, micelle stabilisers, preferably selected from the group consisting of carnitine or/and a physiologically tolerable carnitine derivative or/and polyol(s), or/and radical scavengers, preferably selected from the group consisting of ascorbic acid or/and alkali metal or/and alkaline earth metal salts of ascorbic acid or/and esters of one or more organic acids with ascorbic acid.

6. The composition according to any one of the preceding claims, wherein the composition is prepared as a tablet, a chewable tablet, a prolonged-release tablet, a capsule, a coated tablet, a powder, a granulate, a lyophilisate, a suspension or as an aqueous or oily solution, emulsion, nanoemulsion, nanodispersion system.

7. The composition according to any one of the preceding claims, wherein the lectin is present emulsified in vesicles, mixed micelles, as a microemulsion, as a nanodisperse system or nanoemulsion, or as a liquid, aqueous, micellar emulsion or as a liquid, aqueous-oily, micellar emulsion.

8. The composition according to claim 7, wherein the micelles have an average diameter of 3 to 300 nm, preferably of 5 to 250 nm, particularly preferably of 10 to 120 nm, more preferably of 50 to 80 nm.

9. The composition according to claim 7 or 8, wherein the emulsion of the lectin composition has a pH of from 4 to 9.5, preferably from 6 to 8.5, preferably from 8.5 to 9.5.

10. The composition according to any one of the preceding claims, wherein the composition is for oral administration.

11. The composition according to any one of the preceding claims a. for use in the prophylaxis or/and treatment of (AHT) aromatase inhibitor-induced, anti-oestrogen-induced and/or GnRH analogue-induced adverse drug reactions comprising drying of the entire mucosal complex comprising mucous membranes of the eyes, nose, mouth, throat, vagina and gastrointestinal tract, joint pain, in particular morning stiffness and hot flashes, or b. for use in the prophylaxis or/and treatment of chemo- or radiotherapy-induced adverse drug reactions comprising nausea, vomiting/diarrhoea, dry mucous membranes, joint discomfort, fatigue/weakness, dry mouth, or c. for use in the prophylaxis or/and treatment of natural hormone deficiency due to the menopause, including hot flashes, dry eyes, dryness of the vaginal mucosa and joint problems and an increased rate of infections in the urogenital area, weight gain, excessive nervousness and sensitivity, fatigue, psychological problems such as anxiety, sleep disorders and depressive states, or d. for use in the prophylaxis or/and treatment of adverse drug reactions caused by growth receptor inhibitors, protein inhibitors or immunotherapeutic agents, including drying of the entire mucous membrane complex comprising the mucous membranes of the eyes, nose, mouth, throat, vagina and gastrointestinal tract, joint pain, in particular morning stiffness and hot flashes, or nausea, vomiting/diarrhoea, dry mucous membranes, joint discomfort, fatigue/weakness, dry mouth or hot flashes, dry eyes, dryness of the vaginal mucous membranes and joint problems and an increased rate of infections in the urogenital area, weight gain, excessive nervousness and sensitivity, fatigue, psychological problems such as anxiety, sleep disorders and depressive states.

12. A composition for use according to claim 11, wherein the growth receptor inhibitor is selected from the group consisting of Intracellular Tyrosine Kinase Inhibitors and Extracellular Receptor Inhibitors, the protein inhibitor is selected from the group consisting of AKT Inhibitors, ALK Inhibitors, Angiogenesis Inhibitors, BCR-ABL Inhibitors, B-Raf inhibitors, CDK inhibitors, Hedgehog signaling inhibitors, IGF pathway inhibitors, cMET inhibitors, MEK inhibitors, mTOR inhibitors, PARP inhibitors, PI3K & PIK3CA inhibitors and (tyrosine) kinase inhibitors, or the immunotherapeutic agent is selected from the group consisting of immune checkpoint inhibitors and immunomodulators.

## Revendications

**1.** Composition comprenant au moins un extrait de lentille contenant de la lectine, dans laquelle la teneur en lectine de lentille (lens culinaris) est de 0,5 à 0,08 mg et éventuellement d'autres additifs et adjuvants, et dans laquelle la composition est une composition pharmaceutique, un aliment diététique, un aliment pour régime équilibré, un complément alimentaire ou un dispositif médical.

**2.** Composition selon la revendication 1, ladite composition comprenant en outre au moins un composé de sélénium et/ou au moins une enzyme protéolytique ou/et de la vitamine D ou/et de la vitamine B2 ou/et du zinc ou/et de la curcumine.

**3.** Composition selon l'une quelconque des revendications précédentes, dans laquelle le sélénium est un sélénite de sodium, un sélénite de potassium, ou un sélénite de magnésium, de préférence dans laquelle la dose journalière du composé de sélénium est d'au moins 25-500 µg, de préférence 50-300 µg, du composé de sélénium.

**4.** Composition selon l'une quelconque des revendications précédentes, dans laquelle l'enzyme protéolytique est une papaïne, une bromélaïne, une trypsine ou/et une chymotrypsine, et dans laquelle de préférence la dose journalière des enzymes protéolytiques est d'au moins 10-1000 mg ou 50-5000 unités FIP, de préférence 20-1000 mg ou 100-5000 unités FIP.

**5.** Composition selon l'une quelconque des revendications précédentes, dans laquelle les autres additifs et adjuvants sont choisis dans le groupe constitué par les phospholipides, de préférence les phosphoglycérides, les stabilisants de micelles, de préférence choisis dans le groupe constitué par la carnitine ou/et un dérivé de carnitine physiologiquement acceptable ou/et un ou des polyols, ou/et des capteurs de radicaux, de préférence choisis dans le groupe constitué par l'acide ascorbique ou/et les sels alcalins ou/et alcalino-terreux de l'acide ascorbique ou/et les esters d'un ou plusieurs acides organiques avec l'acide ascorbique.

**6.** Composition selon l'une quelconque des revendications précédentes, ladite composition étant préparée sous la forme d'un comprimé, d'un comprimé à croquer, d'un comprimé à effet retard, d'une gélule, d'une dragée, d'une poudre, d'un granulé, d'un lyophilisat, d'une suspension ou sous la forme d'une solution aqueuse ou huileuse, d'une émulsion, d'une nano-émulsion, d'un système de nano-dispersion.

**7.** Composition selon l'une quelconque des revendications précédentes, dans laquelle la lectine est présente sous forme émulsifiée dans des vésicules, des micelles mixtes, sous forme de microémulsion, de système nanodispersé ou de nanoémulsion, ou sous forme d'émulsion micellaire liquide aqueuse ou d'émulsion micellaire liquide aqueuse/huileuse.

**8.** Composition selon la revendication 7, dans laquelle les micelles ont un diamètre moyen de 3 à 300 nm, de préférence de 5 à 250 nm, de manière particulièrement préférée de 10 à 120 nm, de manière plus préférée de 50 à 80 nm.

**9.** Composition selon la revendication 7 ou 8, dans laquelle l'émulsion de la composition de lectine a un pH de 4 à 9,5, de préférence de 6 à 8,5, de préférence de 8,5 à 9,5.

**10.** Composition selon l'une quelconque des revendications précédentes, ladite composition étant destinée à être administrée par voie orale.

**11.** Composition selon l'une quelconque des revendications précédentes a. pour l'utilisation dans la prophylaxie ou/et le traitement d'effets secondaires indésirables de médicaments induits par les inhibiteurs de l'aromatase (AHT), les antioestrogènes ou/et les analogues de la GnRH, y compris le dessèchement de l'ensemble du complexe muqueux comprenant les muqueuses oculaires, nasales, buccales, pharyngées, vaginales ainsi que celles du tractus gastrointestinal, les douleurs articulaires, en particulier les douleurs de démarrage matinales et les bouffées de chaleur, ou b. pour l'utilisation dans la prophylaxie ou/et le traitement des effets secondaires de médicaments induits par la chimiothérapie ou la radiothérapie, y compris les nausées, les vomissements/diarrhées, la sécheresse des muqueuses, les troubles articulaires, la fatigue/faiblesse, la sécheresse buccale ou, c. pour l'utilisation dans la prophylaxie ou/et le traitement du déficit hormonal naturel induit par la ménopause, y compris les bouffées de chaleur, la sécheresse oculaire, la sécheresse de la muqueuse vaginale ainsi que les problèmes articulaires et un taux accru d'infections dans la région urogénitale, la prise de poids, la nervosité excessive ainsi que la sensibilité, la fatigue, les problèmes psychiques tels que l'anxiété, les troubles du sommeil et les états dépressifs, ou d. pour l'utilisation dans la prophylaxie ou/et le traitement d'effets indésirables de médicaments induits par les inhibiteurs de récepteurs de croissance ou par les inhibiteurs de protéines ou d'effets indésirables de médicaments induits par les immunothérapies, y compris le dessèchement de l'ensemble du complexe muqueux comprenant les muqueuses oculaires, nasales, buccales, pharyngées et vaginales ainsi que celles du tractus gastro-intestinal, les douleurs articulaires, en particulier les douleurs de démarrage matinales et les bouffées de chaleur, ou les nausées, les vomissements/diarrhées, la sécheresse des muqueuses, les troubles articulaires, fatigue/faiblesse, la sécheresse de la bouche ou bouffées de chaleur, la sécheresse oculaire, la sécheresse de la muqueuse vaginale et les problèmes articulaires et un taux accru d'infections dans la zone urogénitale, la prise de poids, la nervosité excessive ainsi que la sensibilité, la fatigue, les problèmes psychiques tels qu'anxiété, les troubles du sommeil et les états dépressifs.

**12.** Composition pour l'utilisation selon la revendication 11, dans laquelle l'inhibiteur de récepteur de croissance est choisi dans le groupe constitué par les inhibiteurs de tyrosine kinase intracellulaire et les inhibiteurs de récepteur extracellulaire, l'inhibiteur de protéine est choisi dans le groupe constitué par les inhibiteurs d'AKT, les inhibiteurs d'ALK, les inhibiteurs d'angiogenèse, les inhibiteurs de BCR-ABL, les inhibiteurs de B-Raf, les inhibiteurs de CDK, les inhibiteurs de signalisation Hedgehog, les inhibiteurs de la voie de signalisation IGF, les inhibiteurs de cMET, les inhibiteurs de MEK, les inhibiteurs de mTOR, les inhibiteurs de PARP, les inhibiteurs de PI3K & PIK3CA et les inhibiteurs de (tyrosine) kinase, ou l'agent immunothérapeutique est choisi dans le groupe constitué par les inhibiteurs de points de contrôle immunitaires et les immunomodulateurs.

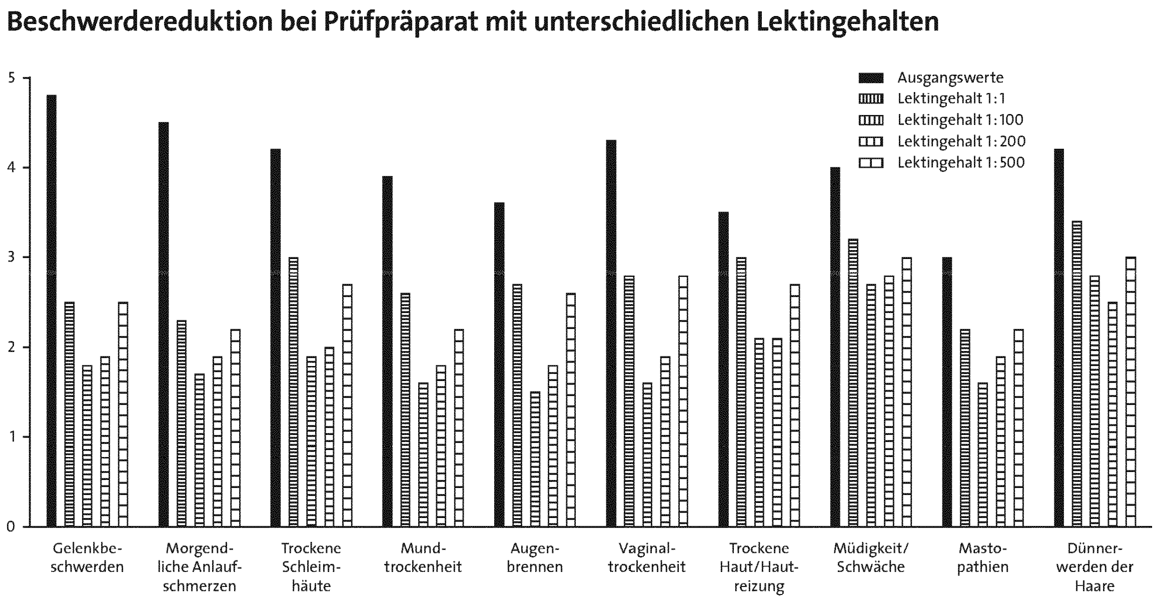
Beschwerdereduktion bei Prüfpräparat mit unterschiedlichen Lektingehalten
Ausgangswerte
Lektingehalt 1:1
Lektingehalt 1:100
Lektingehalt 1:200
Lektingehalt 1:500
0
1
2
3
4
5
Gelenkbe-schwerden
Morgend-liche Anlauf-schmerzen
Trockene Schleim-häute
Mund-trockenheit
Augen-brennen
Vaginal-trockenheit
Trockene Haut/Haut-reizung
Müdigkeit/Schwäche
Masto-pathien
Dünner-werden der Haare

Figur 1

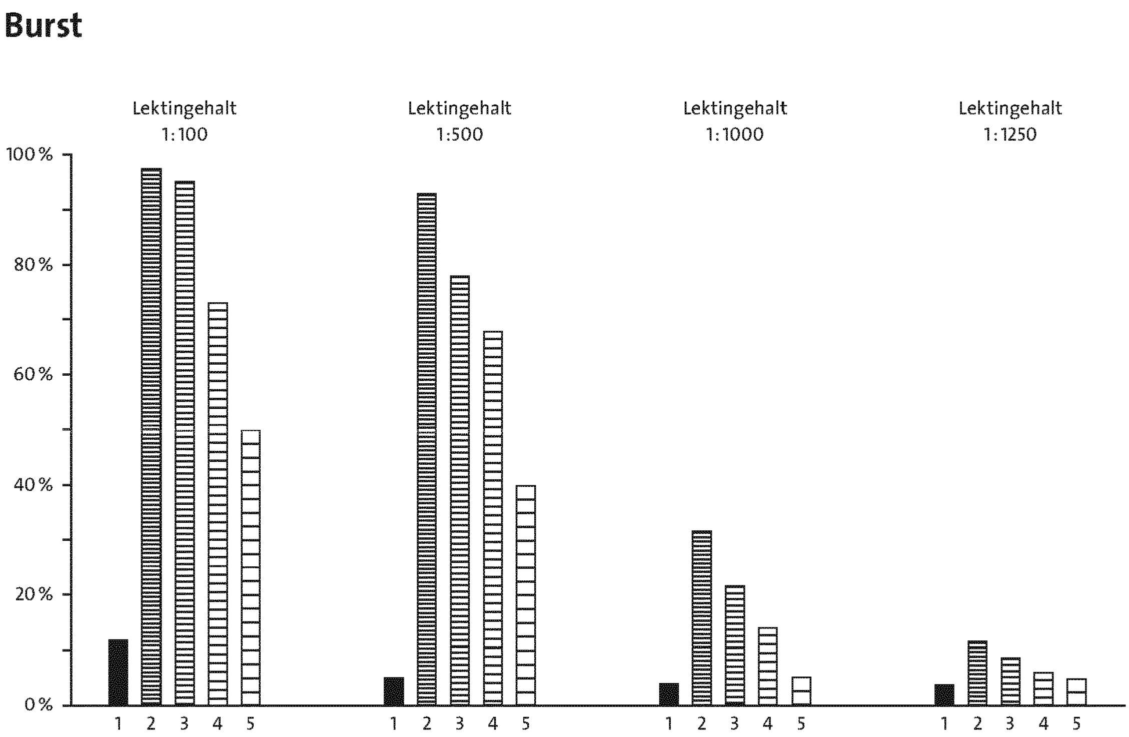
Burst
Lektingehalt 1:100
Lektingehalt 1:500
Lektingehalt 1:1000
Lektingehalt 1:1250
100 %
80 %
60 %
40 %
20 %
0 %
1 2 3 4 5
1 2 3 4 5
1 2 3 4 5
1 2 3 4 5

Figur 2

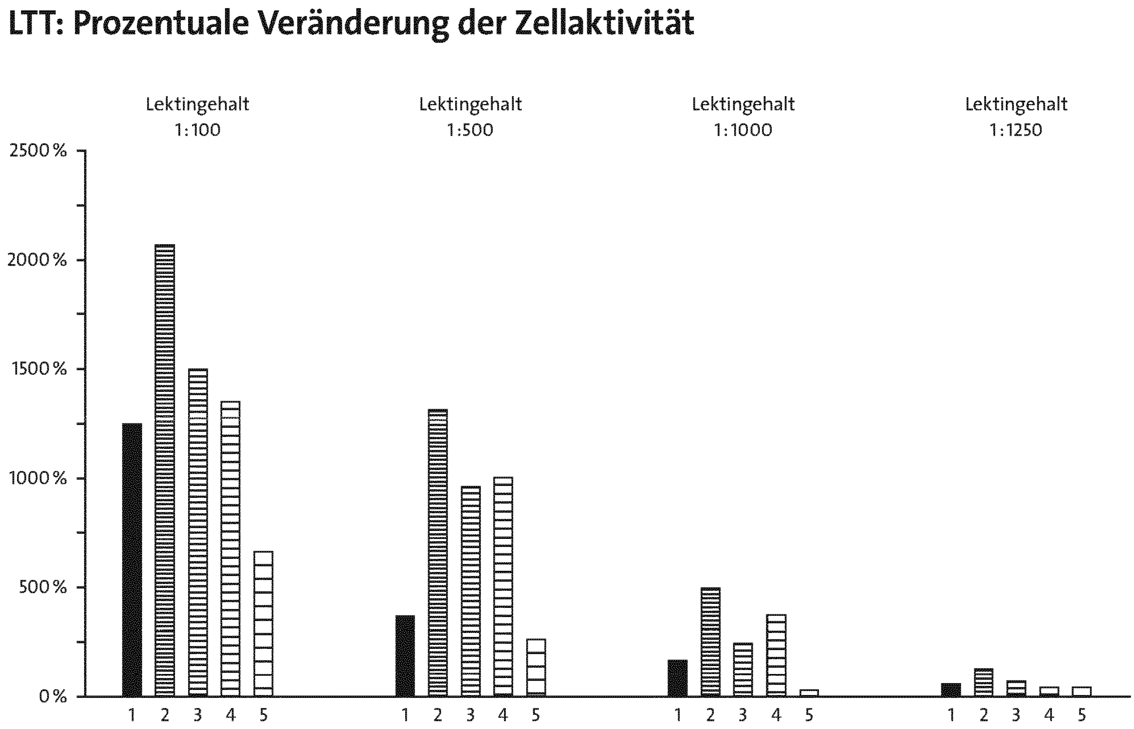
LTT: Prozentuale Veränderung der Zellaktivität
Lektingehalt 1:100
Lektingehalt 1:500
Lektingehalt 1:1000
Lektingehalt 1:1250
2500 %
2000 %
1500 %
1000 %
500 %
0 %
1 2 3 4 5
1 2 3 4 5
1 2 3 4 5
1 2 3 4 5

Figur 3

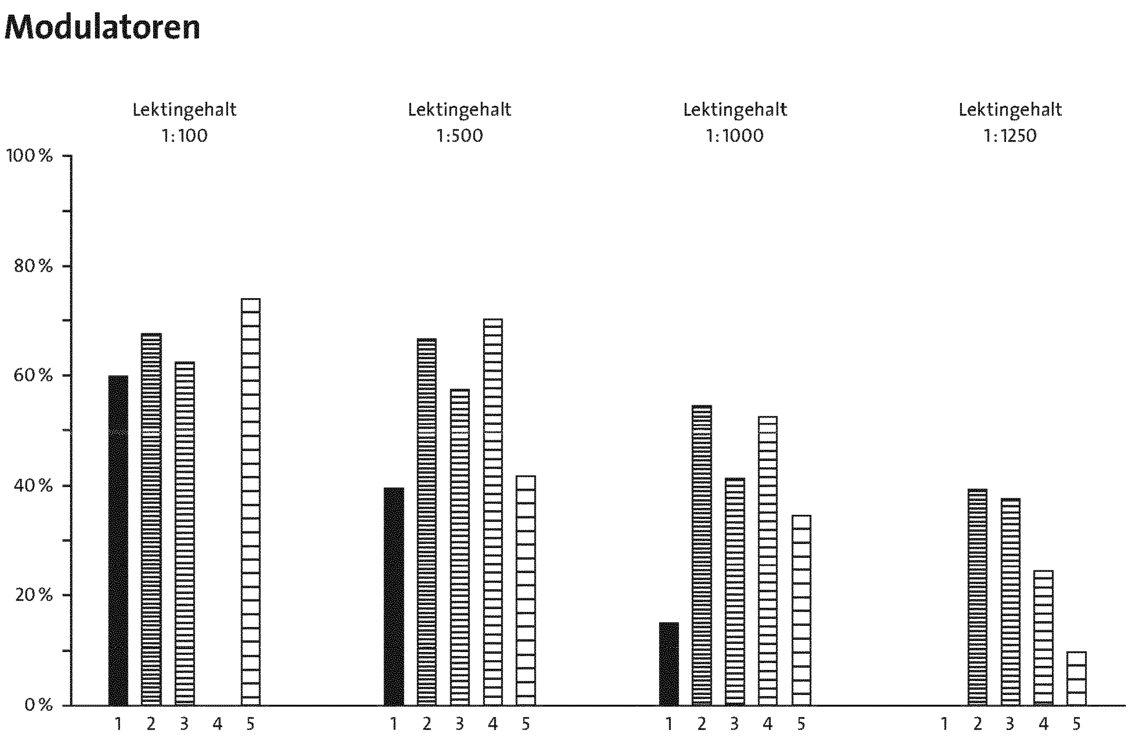
Modulatoren
Lektingehalt 1:100
Lektingehalt 1:500
Lektingehalt 1:1000
Lektingehalt 1:1250
100 %
80 %
60 %
40 %
20 %
0 %
1 2 3 4 5
1 2 3 4 5
1 2 3 4 5
1 2 3 4 5

Figur 4

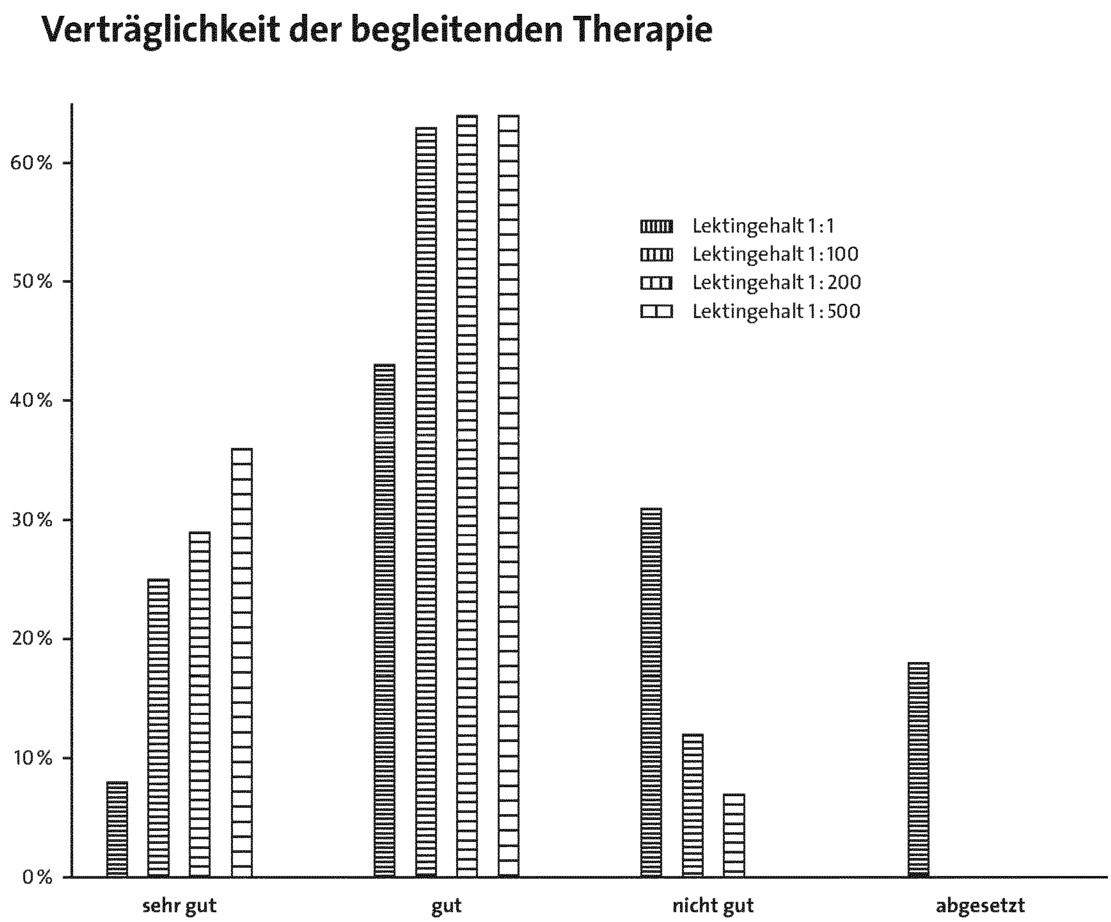
Verträglichkeit der begleitenden Therapie
60%
50%
40%
30%
20%
10%
0%
Lektingehalt 1:1
Lektingehalt 1:100
Lektingehalt 1:200
Lektingehalt 1:500
sehr gut
gut
nicht gut
abgesetzt

Figur 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2106705 B1
- EP 2218456 B1
- DE 102010033458 A1
- EP 2218456 A1


### In der Beschreibung aufgeführte Nicht-Patentliteratur


- **ROITT, I.M.** ; **BROSTOFF, J.** ; **D.K. MALE**. Immunology. Gower Medical Publishing Ltd., 1996
- **SAWYER, D.W.** ; **DONOWITZ, G.R.** ; **G.L. MANDELL**. Polymorphonuclear neutrophils: An effective antimicrobial force.. *Rev. Infect. Dis.*, 1989, vol. 11, S1532-S1544
- Congenital and acquired neutrophil abnormalities. **DONADEBIAN, H. D. et al.** Phagocytes and Disease. Kluwer, 1989, 103-118
- **SMITH, R.M.** ; **J.T. CURNUTTE**. Molecular basis of chronic granulomatous disease. *Blood*, 1991, vol. 77, 673-686
- **ROTHE G** ; **OSER A** ; **G. VALET**. Dihydrorhodamin 123: a new flow cytometric indicator for respiratory burst activity in neutrophil granulocytes. *Naturwissenschaften*, 1988, vol. 75, 354-355
- **MIYAGAWA, B.** ; **H.-G. KLINGEMANN**. Phagocytosis and burst activity of granulocytes and monocytes after stem cell transplantation.. *J. Lab. Clin. Med.*, 1997, vol. 129 (6), 634-7
- **DOBMEYER, T.S.** ; **RAFFEL, B.** ; **DOBMEYER, J.M.** ; **FINDHAMMER, S.** ; **KLEIN, S.A.** ; **KABELITZ, D.** ; **HOELZER, D.** ; **HELM, E.B.** ; **ROSSOL.** Decreased function of monocytes and granulocytes during HIV-1 infection correlates with CD4 cell counts. *Eur. J. Med. Res.*, 1995, vol. 1, 9-15
- **ESPARZA, B.** ; **SÁNCHEZ, H.** ; **RUIZ, M.** ; **BARRANQUERO, M.** ; **SABINO, E.** ; **F. MERINO**. Neutrophil function inelderly persons assessed by flow cytometry. *Immunol. Invest.*, 1996, vol. 25 (3), 185-190
- **GESSLER, P.** ; **NEBE, T.** ; **BIRLE, A.** ; **HAAS, N.** ; **W. KACHEL**. Neutrophil respiratory burst in term and preterm neonates without signs of infection and in those with increased levels of C-Reactive Protein. *Pediatr. Res.*, 1996, vol. 39, 843-848
- **ELBIM, C.** ; **CHOLLET-MARTIN, S.** ; **BAILLY, S.** ; **HAKIM, J.** ; **M.A. GOUGEROT-POCIDALO**. Priming of polymorphonuclear neutrophils by tumor necrosis factor in whole blood: Identification of two polymorphonuclear neutrophil subpopulations in response to formyl-peptides. *Blood*, 1993, vol. 82, 663-640
- **MONIKA-GABRIELE PENZ** ; **A. MÜLLER** ; **S. HOFSTETTER** ; **W. P. BIEGER**. *Laboratoriums Medizin*, June 2002, vol. 26 (5-6), 347-353
- **REICHEL M. et al.** Pilotstudie zum Einfluß von NeyTumorin®-Sol auf Immunparameter von Karzinompatienten. *Naturheilpraxis*, 1998, vol. 5, 2-4
- **KADISH A.S et al.** Natural cytotoxicity and interferon production in human cancer.... *J.Immunol*, vol. 127, 1817
- **BAXEVANIS C. et al.** Mistletoe lectin I induced effects on human cytotoxic lymphocytes. *Immunopharmacology and Immunotoxikology*, 1998, vol. 20 (3), 355-371
- **PENZ M**. Aktuelle Diagnostikverfahren für eine individuell optimierte Tumortherapie mit komplementären Immuntherapeutikum Forum Komplementäre. *Onkologie & Forum Immunologie*, 2000, vol. 1, 14-18
- **HÜLSEN H et al.** Einfluß von Mistelpräparaten auf die in vitro Aktivität der natürlichen. *Killerzellen von Krebspatienten Therapeutikum*, 1993, vol. 7, 434
- **SCHAAF J. et al.** Die natürliche Killerzellenaktivität als wichtiger Parameter bei Immuntherapien mit Biomodulatoren. *Z. Onkol.*, 1996, vol. 28
- **WHITESIDE R. et al.** The role of natural killer cells in immune of cancer. *Current Biol.*
- **MECHELKE F.** *Einfluß von Mistelpräparaten auf die Aktivität der natürlichen Killerzellen im peripheren Blut Uni Hohenheim*
- **RIONDEL J et al.** In vitro comparative study of cytolysis mediated by natural killer cells towards malignant cells preincubated with antioxidants. *Anticancer research*, 1998, vol. 18, 1757-1764
- **IMAI K**. Natural cytotoxic activity of peripheral blood lymphocytes and cancer incidence. *Lancet*, 2000, vol. 356 (25), 1795-1799
- **KIM C.J. et al.** Antitumor Killer Lymphocytes in the peripheral blood of a patient with transitional cell carcinoma of the bladder. *Int. J. Urol*, 1998, vol. 5, 230-236

- **PROSS H.F. et al.** Role of natural killer cells in cancer. *Nat. Immun*, 1993, vol. 12, 279
- **SCHANTZ S.T et al.** Quantitation of natural killer cell function and risk of metastatic poorly differentiated head and neck cancer;. *Nat .Immun Cell growth Regul*, 1991, vol. 10, 278-288
- **CHAKRABORTY A et al.** Age related natural killer activity periperal blood lymphocytes from healthy subjects and cancer patients. *Tumori*, 1994, vol. 80 (3), 233-237
- **KONJEVIC G**. Evaluation of different effects of sera of breast cancer patients on the activity of natural killer cells. *J-Clin. Lab. Immunol.*, 1992, vol. 38 (2), 83-93
- **ONO K**. Clinical significance of natural killing activity in patients with advanced lymphoma. *J. of Clin. Immunol*, 1998, vol. 18, 132-141
- **SHEVDE L.A.** Natural killer cell function and genetic instability in unaffected individuals from breast cancer families. *Europ. J. of Cancer prevention*, 1998, vol. 7, 141-148
- **SCHNELL R ; POYRAZ S ; RADZUHN A ; HARTWIG MT ; DRILLICH S ; SCHÖN G ; MANZKE O ; BOHLEN H ; DIEHL V ; ENGERT A**. Immune response and NK-cell function of Hodgkin's patients treated with an anti-CD25 immunotoxin (RFT5-SMPT-dgA). *Onkologie* (2)
- **J. WRIGHT ; A. YOUNG**. *Lorus Therapeutics, Toronto, Canada*
- **D. BRAUN**. *Stimulation of Natural Killer (NK) Cell Function in Pancreatic Cancer with Virulizin (V), an Immunotherapeutic Agent*